# EUROPÄISCHE PATENTSCHRIFT

(11) **EP 4 440 383 B1**
(45) Veröffentlichungstag und Bekanntmachung des Hinweises auf die Patenterteilung: **12.08.2026**
(21) Anmeldenummer: 21831223.9
(22) Anmeldetag: 30.11.2021
(51) Int. Cl.: A47G 19/22, B65D 90/00

(54) **AUFSATZ FÜR EINE TRINKVORRICHTUNG ZUR RETRONASALEN AUFNAHME EINER AROMASUBSTANZ SOWIE TRINKVORRICHTUNG**
ATTACHMENT FOR A DRINKING DEVICE FOR THE RETRONASAL INTAKE OF AN AROMATIC SUBSTANCE, AND DRINKING DEVICE
EMBOUT POUR DISPOSITIF À BOIRE POUR L'ABSORPTION RÉTRONASALE D'UNE SUBSTANCE AROMATIQUE ET DISPOSITIF À BOIRE

(43) Veröffentlichungstag der Anmeldung: 09.10.2024
(73) Patentinhaber: air up group GmbH, 81671 München (DE)
(72) Erfinder: JÄGER, Tim, 80636 München (DE)
(74) Vertreter: Hoffmann Eitle
(86) Internationale Anmeldenummer: PCT/EP2021/083547
(87) Internationale Veröffentlichungsnummer: WO 2023/098974

(56) Entgegenhaltungen:
- WO-A1-2018/111262
- DE-A1- 102018 222 299
- DE-U1- 202016 004 961

## Beschreibung

### Gebiet der Erfindung

Die Erfindung betrifft einen Aufsatz für eine Trinkvorrichtung zur retronasalen Aufnahme einer Aromasubstanz sowie eine Trinkvorrichtung mit einem derartigen Aufsatz.

### Stand der Technik

Es besteht ein zunehmendes Bedürfnis, Trinkflüssigkeiten aufzunehmen, die einerseits eine angenehme Geschmacksrichtung besitzen, andererseits aber Gesundheitsrisiken zu vermeiden, die durch die Aufnahme von in der Trinkflüssigkeit gelösten Aromasubstanzen oder Stabilisierungsmitteln hervorgerufen werden können. Darüber hinaus soll die Aufnahme einer erhöhten Kalorienmenge vermieden werden.

Daher ist Wasser, das mit einem schwachen Fruchtaroma versehen ist, in den vergangenen Jahren populär geworden. Allerdings finden sich auch in diesem aromatisierten Wasser unerwünschte Additive, wie Stabilisierungssubstanzen und ein gewisser Anteil an Zucker, weshalb diese aromatisierten Getränke ebenfalls eine Kalorienmenge aufweisen, die von vielen Benutzern abgelehnt wird.

Ein erster Schritt zur Lösung des Problems besteht darin, das Geschmacksaroma erst unmittelbar vor dem Konsum eines Getränks diesem zuzuführen. Die US 2008/028353 A1 sowie die US 2015/030726 A1 sind Beispiele für Dosiersysteme, bei denen unmittelbar vor dem Konsum des Getränks oder auch während dessen eine ursprünglich separat vorgesehene Aromasubstanz der Trinkflüssigkeit zugeführt und in dieser gelöst wird. Durch diese Maßnahme können zwar Probleme wie die Stabilisierung der Trinkflüssigkeit über einen verlängerten Zeitraum vermieden werden, jedoch bleibt das Problem der unerwünschten Aufnahme von Zusatzstoffen bestehen.

Da der olfaktorische Sinneseindruck einen wesentlichen Teil der gustatorischen Wahrnehmung beim Konsum von Speisen und Getränken ausmacht, versuchen bisherige Trinksystem den beim Trinken wahrgenommenen Geruch zu beeinflussen. Hierfür wird in der US 5,635,229 ein Aromaelement vorgeschlagen, das an einem Trinkbehälter nahe der Trinköffnung befestigt werden kann, damit sich das Aromaelement in unmittelbarer Nähe zur Nase des Benutzers befindet, der während des Trinkens durch die Nase atmet und somit den Duft aufnimmt. Auch das Trinkgefäß nach der US 8,662,339 B2 arbeitet nach diesem Prinzip, dass während des Trinkens ein Aroma durch die Nase inhaliert wird.

Aus der WO 2019/016096 A1 ist eine Trinkvorrichtung bekannt zur retronasalen Aufnahme einer Aromasubstanz mit einem luftdurchströmbaren Aromabehälter, der aromatisierte Luft einem Transportkanal für Trinkflüssigkeit zuführt. Auf diese Weise wird die Aromasubstanz retronasal aufgenommen. Die Aromasubstanz gelangt beim Trinken gemeinsam mit der Trinkflüssigkeit in den Mund des Benutzers und steigt anschließend retronasal über den Rachenraum zur Riechschleimhaut auf, wo sie durch die dort befindlichen Rezeptoren erfasst und vom Benutzer wahrgenommen wird. Dabei macht man sich den Umstand zunutze, dass ein enger Zusammenhang zwischen dem Geruchssinn und Geschmackssinn besteht. Der Benutzer gewinnt daher den Eindruck, als würde er das Aroma schmecken, obwohl er es tatsächlich lediglich retronasal riecht.

Die WO 2020/126210 A1 beschreibt eine spezielle Ausgestaltung einer derartigen Trinkvorrichtung zur retronasalen Aufnahme einer Aromasubstanz einschließlich der Geometrie eines Trinkhalms mit einem Transportkanal für Trinkflüssigkeit sowie der Geometrie eines im Wesentlichen ringförmigen Aromabehälters.

Die Trinkvorrichtung nach DE 20 2016 004961 U1 stellt den nächstkommenden Stand der Technik **dar.**

### Darstellung der Erfindung

Es besteht ein zunehmendes Bedürfnis, Trinkvorrichtungen zur retronasalen Aufnahme einer Aromasubstanz möglichst variabel im Hinblick auf die aufzunehmenden Trinkflüssigkeiten zu gestalten, aber auch möglichst nachhaltig einzusetzen.

Der Erfindung liegt daher die Aufgabe zugrunde, eine Trinkvorrichtung zur retronasalen Aufnahme einer Aromasubstanz variabler in Bezug auf die Trinkflüssigkeiten sowie nachhaltiger zu gestalten.

Diese Aufgabe wird durch einen Aufsatz für eine Trinkflüssigkeit mit den Merkmalen des Anspruchs 1 sowie eine Trinkvorrichtung zur retronasalen Aufnahme einer Aromasubstanz mit den Merkmalen des Anspruchs 12 gelöst. Bevorzugte Ausführungsformen folgen aus den übrigen Ansprüchen.

Der erfindungsgemäße Aufsatz für eine Trinkvorrichtung zur retronasalen Aufnahme einer Aromasubstanz umfasst ein Adapterelement zur flüssigkeitsdichten Anbringung an einem Vorratsbehälter für Trinkflüssigkeit, ein Kopfteil zur lösbaren Befestigung an dem Adapterelement, und einen luftdurchströmbaren Aromabehälter, der am Kopfteil befestigt ist. Darüber hinaus ist ein Transportkanal für Trinkflüssigkeit am Kopfteil befestigbar. Das Adapterelement umfasst ein erstes Adapterteil sowie ein zweites Adapterteil, wobei das erste Adapterteil zur Anbringung auf dem Vorratsbehälter sowie zur Anbringung an dem zweiten Adapterteil angepasst ist. Das zweite Adapterteil weist eine erste Öffnung mit einer ersten Längsachse sowie eine zweite Öffnung mit einer zweiten Längsachse auf, und die erste Längsachse steht in einem Winkel zur zweiten Längsachse, der mindestens 20° beträgt.

Das Vorsehen eines Adapterelements, das ein erstes Adapterteil sowie in zweites Adapterteil umfasst, macht es möglich, das erste Adapterteil auf unterschiedlichen Vorratsbehältern für Trinkflüssigkeit zu befestigen, da unterschiedlich geformte erste Adapterteile mit dem zweiten Adapterteil zusammenwirken können, so dass die ersten Adapterteile jeweils am zweiten Adapterteil anbringbar sind und für unterschiedliche Anbringegeometrien eines Vorratsbehälters für Trinkflüssigkeit anpassbar sind. Im einfachsten Fall könnte es sich dabei um unterschiedliche Gewindeformen oder Gewindedurchmesser handeln, die an einem Vorratsbehälter für Trinkflüssigkeit vorgesehen sind.

Darüber hinaus bietet das Adapterelement, das ein erstes Adapterteil sowie ein zweites Adapterteil umfasst, den Vorteil, dass sich die Fertigung des Adapterelements vereinfachen lässt, weil die gekröpfte Bauform des Adapterelements mit den winklig zueinander angeordneten ersten und zweiten Längsachsen einen erhöhten Fertigungsaufwand mit sich bringt. Das Herstellen des Adapterelements aus zwei Adapterteilen vereinfacht darüber hinaus auch die Einhaltung von Fertigungstoleranzen.

Weiterhin kann der erfindungsgemäße Aufsatz im Sinne eines modularen Systems auch auf einem Vorratsbehälter für Trinkflüssigkeit angebracht werden, der aus einem beliebigen Material, wie Glas, Stahl oder Kunststoffmaterialien besteht. Insbesondere bei der Verwendung eines Vorratsbehälters für Trinkflüssigkeit aus Stahl weist dieser eine gegenüber einem Aufsatz aus Kunststoffmaterial erhöhte Lebensdauer auf, so dass der Aufsatz austauschbar ist. Ein Austausch des erfindungsgemäßen Aufsatzes kann auch aus hygienischen Gründen gewünscht sein. Indem in diesem Fall nur der Aufsatz, nicht aber die vollständige Trinkvorrichtung ausgetauscht werden muss, leistet der erfindungsgemäße Aufsatz einen Beitrag zur Nachhaltigkeit der mit dem erfindungsgemäßen Aufsatz versehenen Trinkvorrichtung.

Die erste Öffnung des zweiten Adapterteils mit einer ersten Längsachse kann zur lösbaren Befestigung des Kopfteils dienen, während die zweite Öffnung mit einer zweiten Längsachse diejenige Seite des Aufsatzes darstellt, an der das erste Adapterteil anbringbar ist. Die zweite Längsachse entspricht der Längsachse eines mit dem Aufsatz verbindbaren einem Vorratsbehälters für Trinkflüssigkeit.

Die winklige Anordnung der ersten Längsachse zur zweiten Längsachse besitzt den Vorteil, dass das Kopfteil winklig in Bezug auf den Vorratsbehälter für Trinkflüssigkeit angeordnet ist, wodurch eine mit dem erfindungsgemäßen Aufsatz versehene Trinkvorrichtung eine verbesserte Ergonomie besitzt und darüber hinaus für einen Benutzer intuitiv deutlich ist, wie er die Trinkvorrichtung halten soll. Durch das Vorsehen eines Knicks in der Erstreckung des Adapterelements und damit der mit dem Adapterelement verbundenen Trinkvorrichtung wird zudem die Trinkvorrichtung beim Trinken deutlich weniger weit gekippt, wodurch die Gefahr eines unbeabsichtigten Verschüttens von Trinkflüssigkeit verringert wird. Das Vorsehen eines Winkels zwischen der ersten Längsachse und der zweiten Längsachse ermöglicht es auch, den Trinkbehälter in aufrechter Position auszutauschen, ohne den Trinkbehälter kippen zu müssen.

Vorzugsweise umfasst der Aromabehälter einen von einer Behälterwand umschlossenen, inneren Hohlraum, eine Lufteintrittsöffnung durch die Behälterwand und eine Luftaustrittsöffnung durch die Behälterwand.

In dem von der Behälterwand umschlossenen inneren Hohlraum kann auf eine geeignete Weise eine Aromasubstanz enthalten sein. Dazu kann sich in dem inneren Hohlraum des Aromabehälters ein Trägermaterial befinden, das mit der Aromasubstanz oder einer Mischung verschiedener Aromasubstanzen versehen ist. Die Aromasubstanz ist dabei bevorzugt in flüssiger Form vorgesehen.

Ebenfalls bevorzugt ist es, dass sich in dem inneren Hohlraum neben dem mit Aromasubstanz versehenen Trägermaterial auch ein Kopfraum befindet, der mit Luft gefüllt ist und dazu dient, die durch die Lufteintrittsöffnung durch die Behälterwand eingeströmte Luft vor dem Austritt aus der Luftaustrittsöffnung durch die Behälterwand möglichst gleichmäßig mit Aromasubstanz anzureichern.

Die Trägersubstanz in dem inneren Hohlraum des Aromabehälters umfasst ein poröses Aromaträgermaterial, das vorzugsweise Vliesmaterial umfasst. Weiterhin bevorzugt beträgt die Luftdurchlässigkeit des porösen Aromaträgermaterials bei 100 Pa Differenzdruck mindestens 200 l/(m²·s) und bevorzugt zwischen 220 l/(m²·s) und 280 l/(m²·s).

Weiterhin bevorzugt liegt die Porosität des Aromaträgermaterials zwischen 70% und 93% und bevorzugt zwischen 70% und 80%.

Weiterhin bevorzugt ist es, dass die Reynoldszahl Re im Bereich der Lufteintrittsöffnung des Aromabehälters mindestens 2300 beträgt, wobei die dimensionslose Reynoldszahl definiert ist als Re=(w·d)/v mit der kinematischen Viskosität von Luft ν[m²/s], dem Durchmesser d[m] der Lufteintrittsöffnung und der Strömungsgeschwindigkeit w[m/s] der Luft beim Einströmen durch die Lufteintrittsöffnung in den Aromabehälter mit einem gemittelten Volumenstrom zwischen 250 ml/min und 600ml/min.

Eine Reynoldszahl von mindestens 2300 charakterisiert eine turbulente Strömung oder zumindest eine Strömung, die sich bereits im Übergangsbereich zwischen einer laminaren und turbulenten Strömung befindet. Eine turbulente Strömung gewährleistet eine gute Durchmischung, wodurch die Aufnahme der Aromasubstanz in dem den Aromabehälter durchströmenden Luftstrom verbessert wird. Der angegebene Volumenstrom pro Minute berücksichtig die zum Teil erheblichen Schwankungen im temporären Volumenstrom, wenn der erfindungsgemäße Aufsatz mit einem Vorratsbehälter für Trinkflüssigkeit gekoppelt ist und ein Benutzer aus der Trinkvorrichtung trinkt. Da der Trinkvorgang kein gleichmäßiger, stationärer Vorgang ist, sondern im Rahmen der Saug- und Schluckvorgänge unterschiedliche Drücke, aber auch unterschiedliche Strömungsgeschwindigkeiten im Transportkanal für Trinkflüssigkeit herrschen, besteht eine beträchtliche Fluktuation der temporären Volumenströme und damit auch Strömungsgeschwindigkeiten, mit denen die Luft durch die Lufteintrittsöffnung hindurchtritt. Experimentell konnte jedoch gezeigt werden, dass ein erfindungsgemäßer Aufsatz mit einem Aromabehälter bei seinem bestimmungsgemäßen Einsatz mit einem gemittelten Volumenstrom zwischen 250 ml/min und 600 ml/min durchströmt wird. Der physiologische Trinkvorgang wurde dabei an zahlreichen, unterschiedlichen, erwachsenen Personen ermittelt. Da der Volumenstrom das Produkt aus Strömungsgeschwindigkeit und Querschnittsfläche ist, kann unter der Vorgabe, dass die Strömungszustand beschreibende Reynoldszahl mindestens 2300 betragen muss, ein geeigneter Bereich für den Öffnungsquerschnitt der Lufteintrittsöffnung bestimmen.

Nach einer bevorzugten Ausführungsform der Erfindung ist das zweite Adapterteil zumindest bereichsweise mehrwandig, insbesondere doppelwandig.

Diese Gestaltung hat den Vorteil, dass die Wärmeisolierung des Adapterteils deutlich verbessert wird und sich das Adapterteil auf diese Weise auch dazu eignet, mit einem Vorratsbehälter für Trinkflüssigkeit verwendet zu werden, der mit Trinkflüssigkeit gefüllt ist, die über einen verlängerten Zeitraum kalt oder heiß bleiben soll. Besonders geeignet ist das doppelwandige Adapterteil zur Verwendung auf einem Vorratsbehälter für Trinkflüssigkeit, der ein Thermosgefäß darstellt.

Nach einer bevorzugten Ausgestaltung eines doppelwandig gestalteten Adapterteils ist in einem Raum zwischen einer Innenwand und einer Außenwand des zweiten Adapterteils ein UV-Licht emittierendes Bauelement inklusive Aggregate, wie beispielsweise Batterien und Prozessoren platzierbar. Ein UV-Licht emittierendes Bauelement beispielsweise in Form eine UV-Diode kann dabei so angeordnet werden, dass diese im Betrieb durch die Innenwand des zweiten Adapterteils den von der Innenwand des Adapterteils umschlossenen Raum desinfiziert. Ebenso können Sensorelemente verbaut und in den Adapter integriert werden, wie beispielsweise eine Füllstandsmessung, die über eine kabellose Datenverbindung mittels NFC mit einem Smartdevice gekoppelt werden kann. Ein Vorteil der Integration von Funktionselementen in den Adapter ist neben der Modularität des Systems vor allem auch ein schneller, kostengünstiger und nachhaltiger Austausch der einem schnellen technischen Fortschritt unterliegenden Sensortechnik.

Nach einer bevorzugten Ausführungsform der Erfindung weist das Kopfteil einen Luftkanal auf, dessen zweites Ende in Strömungsverbindung mit einem Innenraum im Transportkanal für Trinkflüssigkeit steht, und dessen erstes Ende so angeordnet ist, dass der Aromabehälter in eine Position bewegbar ist, in der die Luftaustrittsöffnung des Aromabehälters mit dem ersten Ende des Luftkanals in Strömungsverbindung steht.

Auf diese Weise kann die den Aromabehälter durchströmende Luft unmittelbar beim Austritt aus der Luftaustrittsöffnung des Aromabehälters in den Luftkanal eintreten, der in den Transportkanal für Trinkflüssigkeit mündet. Das Merkmal, wonach der Aromabehälter in eine Position bewegbar ist, in der die Luftaustrittsöffnung mit dem ersten Ende des Luftkanals in Strömungsverbindung steht, impliziert in gleicher Weise aber auch, dass der Aromabehälter auch in eine Position gebracht werden kann, in der diese Strömungsverbindung nicht besteht und somit keine Luft aus dem Aromabehälter austreten kann. In dieser Position gelangt keine aromatisierte Luft in den Transportkanal für Flüssigkeit und der Aromabehälter befindet sich in einer Ruheposition, in der der Austritt von aromatisierter Luft verhindert wird oder zumindest weitestgehend verhindert wird.

Nach einer bevorzugten Ausführungsform der Erfindung besitzt der Transportkanal für Trinkflüssigkeit eine Längsachse, die einen gekrümmten Verlauf hat.

Nachdem erfindungsgemäß der Aufsatz ein Adapterelement umfasst, dessen zweites Adapterteil gekröpft ist, da die erste Längsachse in einem Winkel zur zweiten Längsachse steht, und der Transportkanal für Trinkflüssigkeit am Kopfteil befestigbar ist, besitzt der gekrümmte Verlauf des Transportkanals den Vorteil, dass dieser bis nahe an den Boden eines Vorratsbehälters für Trinkflüssigkeit geführt werden kann, so dass bei der Verwendung des erfindungsgemäßen Aufsatzes auf einem Vorratsbehälter mit Trinkflüssigkeit mit geeigneter Geometrie die Trinkflüssigkeit nahezu vollständig vom Benutzer aufgesogen werden kann.

Dabei ist es vorteilhaft, den Transportkanal für Trinkflüssigkeit als Trinkhalm auszugestalten, dessen Außenquerschnitt zumindest im Bereich der Befestigung am Kopfteil so gestaltet ist, dass der Trinkhalm nur in einer einzigen Rotationsposition in Bezug auf die Längsachse am Kopfteil befestigbar ist. Das Kopfteil ist bevorzugt aus Silikon gefertigt und kann den eingeschobenen Trinkhalm reibschlüssig halten.

Der Vorteil dieser Gestaltung besteht darin, dass durch die Definition der Rotationsposition bei der Befestigung am Kopfteil auch die Krümmung im Verlauf der Längsachse des Transportkanals eine definierte Orientierung aufweist, die auf die Gesamtgeometrie des Aufsatzes für eine Trinkvorrichtung bei dessen Anbringung auf einem Vorratsbehälter für Trinkflüssigkeit abgestimmt ist.

Vorzugsweise ist der Aromabehälter im Wesentlichen ringförmig mit einer radial äußeren Begrenzungswand und einer radial inneren Begrenzungswand, wobei die radial innere Begrenzungswand einen Raum umschließt, dessen Querschnittsfläche eine von einer kreisrunden Form abweichende Geometrie besitzt.

Das Vorsehen eines Aromabehälters mit einer im Wesentlichen ringförmigen Geometrie besitzt zahlreiche Vorteile. Zum einen lässt sich ein derartiger Aromabehälter um den Transportkanal für Trinkflüssigkeit am Kopfteil des Aufsatzes befestigen, ohne dass das Gewicht des Aromabehälters bei der Verwendung des Aufsatzes auf einem Vorratsbehälter für Trinkflüssigkeit als störend empfunden wird, weil es zu keiner unsymmetrischen Gewichtsverteilung an der Trinkvorrichtung kommt. Darüber hinaus kann durch das Vorsehen einer ringförmigen Geometrie durch eine gezielte Anordnung der Lufteintrittsöffnung und der Luftaustrittsöffnung sichergestellt werden, dass der den Aromabehälter durchströmende Luftstrom einen möglichst langen Weg zurücklegt und sich dabei möglichst vollständig mit der in der Aromakammer befindlichen Aromasubstanz anreichert.

Indem die radial innere Begrenzungswand einen Raum umschließt, dessen Querschnittsfläche eine von einer kreisrunden Form abweichende Geometrie besitzt, kann das korrekte Platzieren des Aromabehälters an dem Aufsatz erleichtert werden. Wenn dabei die von einer kreisrunden Form abweichende Geometrie nur eine einzige Position des Aromabehälters in Bezug auf seine Rotation relativ zum Kopfteil ermöglicht, lässt sich eine eindeutige Positionierung des Aromabehälters relativ zum Kopfteil sicherstellen. Außerdem wird die den Aromabehälter durchströmende Luft durch Querschnittsänderungen und Änderungen der Krümmung des inneren Hohlraums der Aromakammer besser durchmischt, weil sämtliche Änderungen im Strömungsverlauf das Auftreten von Turbulenzen unterstützen.

Nach einer bevorzugten Ausführungsform der Erfindung weist das Adapterelement eine Aufnahmeeinrichtung zur Anbringung einer Trageschlaufe auf. Die Aufnahmeeinrichtung kann dabei beispielsweise als Nut-Feder-System ausgestaltet sein.

Auf diese Weise lassen sich unterschiedliche Trageschlaufen bequem am Adapterelement befestigen. Durch die Wahl der Farbe der Trageschlaufe lässt sich der Aufsatz nach Wunsch des Benutzers individualisieren, aber auch bei der Verwendung unterschiedlicher erster Adapterteile zur Anbringung an unterschiedliche Vorratsbehälter für Trinkflüssigkeit eine für einen Benutzer einfach zu erfassende Differenzierung erzielen.

Durch die Anordnung der Trageschlaufe an dem zweiten Adapterteil so, dass die Trageschlaufe in demjenigen Bereich des zweiten Adapterteils angeordnet ist, in dem dessen Längserstreckung mit der Längserstreckung des Vorratsbehälters übereinstimmt, kann die Trageschlaufe auch während des Trinkprozesses verwendet werden. Darüber hinaus befindet sich die Trageschlaufe an einem Teil der Trinkvorrichtung, die beim Auf- und Abschrauben des Deckels 22 nicht rotiert, so dass ein Benutzer die Trinkvorrichtung an der Trageschlaufe halten kann, während der Deckel abgeschraubt oder aufgeschraubt wird.

Da die Trageschlaufe abnehmbar ist, kann diese im Falle einer Beschädigung leicht ersetzt werden. Darüber hinaus kann die Trageschlaufe als Interface zu einer Energiespeichereinrichtung, wie einer Batterie oder einem Akkumulator dienen, um beispielsweise eine UV-Strahlung emittierende Baueinheit, die eine zugehörige Energiespeichereinrichtung benötigt, mit Energie versorgen zu können.

Durch die Formgebung des zweiten Adapterteils mit der abgeknickten Form kann ein Benutzer bequem aus dem Mundstück trinken, ohne dass die Trinkvorrichtung dabei insgesamt gekippt werden muss. Die Trinkvorrichtung kann dabei mit Hilfe der Trageschlaufe in einer aufrechten Position, **d.h.** mit dem Boden in horizontaler Ausrichtung benutzt werden, oder aber die Trinkvorrichtung kann während des Trinkvorgangs auf einer horizontalen Oberfläche stehen.

Vorzugsweise bestehen das Adapterelement sowie das Kopfteil aus Kunststoff. Durch die Verwendung von Kunststoff lässt die das Adapterelement einfach im Rahmen einer Massenfertigung durch Spritzguss herstellen. Darüber hinaus kann durch eine geeignete Farbwahl des eingesetzten Kunststoffmaterials zusätzlich zur Farbwahl der Trageschlaufe eine weitere Individualisierung und farbliche Abstimmung mit der Trageschlaufe erzielt werden. Kunststoffe sind besonders geeignet, da sie eine geringe Dichte aufweisen und daher die Herstellung in Leichtbauweise ermöglichen, was neben einer Materialeinsparung und geringeren Transportkosten auch von den Benutzern als angenehm empfunden wird. Außerdem lassen Kunststoffe eine erhöhte Gestaltungsfreiheit zu durch die Verarbeitung als Schmelze im Spritzgussverfahren. Dies ist nicht nur vorteilhaft für die Herstellung komplexer Geometrien in Integralbauweise, bei der die Funktionalitäten mehrerer Bauteile in einem einzigen Bauteil vereint werden, sondern auch zur Reduzierung zugehöriger Fertigungsschritte.

Nach einer bevorzugten Ausführungsform weist das zweite Adapterteil im Bereich der ersten Öffnung mindestes ein erstes Positionierelement auf, das mit einem zweiten Positionierelement zusammenwirkt, das am Kopfteil vorgesehen ist, so dass das Kopfteil nur einer definierten Position relativ zur ersten Längsachse an dem zweiten Adapterelement anbringbar ist.

Durch das Vorsehen des mindestens einen ersten Positionierelements kann das Kopfteil zentriert werden. Dies ist aufgrund der gekröpften Form des Aufsatzes erforderlich, weil im Kopfteil ein Druckausgleichskanal vorgesehen ist, der während des Trinkvorganges am oberen Ende der Trinkvorrichtung angeordnet sein sollte, damit beim Trinkvorrichtung keine Trinkflüssigkeit austreten kann. Darüber hinaus kann ein Transportkanal für Trinkflüssigkeit, dessen Längsachse einen gekrümmten Verlauf besitzt nur dann korrekt verwendet werden, wenn das Kopfteil richtig eingesetzt wurde, **d.h.** das Kopfteil in korrekter Orientierung eingesetzt wurde. Das erste Positionierelement und das zweite Positionierelement sind dabei bevorzugt eine oder mehrere Rippen und eine oder mehrere Vertiefungen, die beim Anbringen des Kopfteils am zweiten Adapterelement auch eine Führungsfunktion erfüllen, wodurch auch das Anbringen des Kopfteils am Adapterelement erleichtert wird.

Die erfindungsgemäße Trinkvorrichtung besteht vorzugsweise aus einem anderen Material als das Adapterelement, wobei vorzugsweise das Adapterelement aus Kunststoff und der Vorratsbehälter aus Metall besteht.

Auf diese Weise lässt sich die unterschiedliche Lebenszeit der einzelnen modularen Komponenten berücksichtigen oder aber eine Beschädigung entweder des Vorratsbehälters für Trinkflüssigkeit oder aber des Aufsatzes durch den gezielten Austausch nur der beschädigten Komponente beseitigen, so dass die erfindungsgemäße Trinkvorrichtung über einen langen Zeitraum nachhaltig eingesetzt werden kann.

Vorzugsweise ist der Vorratsbehälter für Trinkflüssigkeit zumindest bereichsweise doppelwandig. Auf diese Weise kann der Vorratsbehälter sowohl dazu verwendet werden, warme wie auch kalte Trinkflüssigkeit möglichst lange kalt oder warm zu halten. Darüber hinaus leistet eine doppelschalige Struktur mit geringerem Materialeinsatz dieselbe mechanische Stabilität wie ein einwandiger Vorratsbehälter. Auf diese Weise kann bei der Massenfertigung der erfindungsgemäßen Trinkvorrichtungen eine erhebliche Materialeinsparung erzielt werden.

Auch im Bereich der Verbindung zwischen dem ersten Adapterteil und dem zweiten Adapterteil kann ein geeignetes Positionier- und Führungselement vorgesehen sein, beispielsweise in Form einer Führungsrippe an der dem ersten Adapterteil zugewandten Wandung des zweiten Adapterteils, um das Anbringen des ersten Adapterteils an dem zweiten Adapterteil zu erleichtern, indem das erste Adapterteil nur in einer definierten Lage relativ zum zweiten Adapterteil in dieses eingeschoben werden kann. Darüber hinaus kann eine formschlüssig wirkende Führungs- und Positioniereinrichtung zwischen dem ersten Adapterteil und dem zweiten Adapterteil auch dahingehend wirken, dass das zweite Adapterteil nicht relativ zum ersten Adapterteil rotiert werden kann, so dass ein Benutzer durch eine Drehung des zweiten Adapterteils relativ zum Vorratsbehälter für Trinkflüssigkeit automatisch ein auf den Vorratsbehälter für Trinkflüssigkeit aufgeschraubtes erstes Adapterteil von dem Vorratsbehälter lösen oder aber auf den Vorratsbehälter aufschrauben kann.

Nach einer bevorzugten Ausführungsform der Erfindung weist das Kopfteil eine Luftkammer in Verbindung mit dem Luftkanal auf. Diese Luftkammer stellt einen Sicherheitspuffer dar, um Überdrücke und Unterdrücke während und nach dem Trinken abzupuffern, wodurch vermieden werden kann, das Trinkflüssigkeit in den Aromabehälter strömen kann. Dies löst insbesondere das Problem, dass die bei Beendigung des Trinkvorgangs an der Trinkvorrichtung auftretenden Schwankungen der Druck- und Strömungsbedingungen in dem Transportkanal für Trinkflüssigkeit sowie dem Luftkanal im Kopfteil dazu führen können, dass es zu einem Eintreten von Trinkflüssigkeit in den Luftkanal und/oder dem Aromabehälter kommen kann. Ein Eintreten von Flüssigkeit in den Aromabehälter könnte dazu führen, dass die Aromasubstanz ungewünscht verdünnt wird oder es zu hygienischen Problemen kommt. Die Unterbrechung des Luftkanals durch eine zwischengeschaltete Luftkammer kann dahingehend ausgeführt sein, dass an der Kontaktstelle des Transportkanals für Trinkflüssigkeit und des Luftkanals durch das Kopfteil eine Aussparung am Kopfteil der Trinkvorrichtung vorgesehen ist. Wenn die Luftkammer ringförmig um den Transportkanal für Trinkflüssigkeit herumläuft, besitzt dies den zusätzlichen Vorteil, dass der Transportkanal für Trinkflüssigkeit in einer beliebigen Rotationsposition in Bezug auf die Längsachse am Kopfteil befestigbar ist, da ein Lufteintrittskanal in den Transportkanal für Trinkflüssigkeit in der Luftkammer endet und somit die radiale Orientierung auch nicht auch den Luftkanal im Kopfteil abgestimmt werden muss.

Der wesentliche Gesichtspunkt des erfindungsgemäßen Aufsatzes für eine Trinkvorrichtung besteht darin, dass der Aufsatz auf unterschiedliche Vorratsbehälter für Trinkflüssigkeit aufsetzbar ist und unabhängig von dem Material und der Anschlussgeometrie des Vorratsbehälters für Trinkflüssigkeit durch eine geeignete Gestaltung des ersten Adapterteils anpassbar ist.

Der Vorratsbehälter für Trinkflüssigkeit kann dabei mit reinem Wasser gefüllt werden, in gleicher Weise aber auch mit Kohlensäure versetztes Wasser oder aber eine Trinkflüssigkeit mit Eigengeschmack aufnehmen. Bei der Verwendung einer Trinkflüssigkeit mit Eigengeschmack kann dabei der bestehende Eigengeschmack der Trinkflüssigkeit durch die Aromasubstanz aus dem Aromabehälter entweder verstärkt oder aber durch eine oder mehrere zusätzliche Geschmackskomponenten ergänzt werden.

Nach einer bevorzugten Ausführungsform der Erfindung ist der austauschbare Aromabehälter in einer axialen Richtung von einer abdichtenden Position in eine nicht abdichtende Position relativ zum Kopfteil bewegbar. Auf diese Weise kann mit Hilfe einer einfachen axialen Bewegung des Aromabehälters dieser von einer abdichtenden Position in eine Betriebsposition bewegt werden, in der der Aromabehälter mit Luft durchströmbar ist. Wenn in der abdichtenden Position die Lufteintrittsöffnung des Aromabehälters geschlossen ist, lässt sich die Verflüchtigung der Aromasubstanz in die Umwelt sowie das Eindringen von Flüssigkeit in den Aromabehälter verhindern.

Der erfindungsgemäße Aufsatz umfassend das Adapterelement kann in Form eines modularen Systems auf unterschiedliche Vorratsbehälter mit unterschiedlichen Anschlussmaßen und aus unterschiedlichen Materialien aufgesetzt werden. Der Vorratsbehälter kann einfach befüllt und wieder befüllt werden, da der gesamte Aufsatz lediglich vom Vorratsbehälter gelöst werden muss, während der gesamte Aufsatz mit Deckel und Trinkhalm in Betriebsposition verbleiben kann.

Da ein Vorratsbehälter für Trinkflüssigkeit beispielsweise aus Metall erfahrungsgemäß eine höhere Lebensdauer besitzt als ein Aufsatz aus Kunststoffmaterial, kann durch den Austausch des Aufsatzes die Trinkvorrichtung wieder weiterverwendet werden und damit möglichst nachhaltig verwendet werden.

Durch die Formgebung des Aufsatzes, der im Bereich des ersten Adapterteils vom Vorratsbehälter für Trinkflüssigkeit gelöst werden kann, lässt sich die Einfüllöffnung der Trinkvorrichtung so anordnen, dass diese möglichst nahe an dem maximalen Füllstand mit Trinkflüssigkeit angeordnet ist, wodurch ein Wiederbefüllen der Trinkvorrichtung beispielsweise unter einem Wasserhahn bei verringertem Platzangebot erleichtert wird. In gleicher Weise kann aber auch bei beschränktem Raumangebot zwischen dem Auslass eines Wasserhahns und einem Waschbecken die Trinkvorrichtung befüllt werden, indem das Kopfteil entfernt wird und Wasser durch die erste Öffnung im zweiten Adapterteil 18 nachgefüllt wird, während die Längsachse des Vorratsbehälters zur Vertikalen geneigt ist.

Die Trinkvorrichtung kann beim Trinken verwendet werden, ohne dass dabei die Trinkvorrichtung entweder festgehalten oder manuell in eine bestimmte Position gebracht werden muss, so dass diese auch für Personen mit körperlichen Einschränkungen leicht verwendbar ist.

### Kurze Beschreibung der Zeichnungen

In den Figuren sind beispielhafte Ausführungsformen der Erfindung dargestellt. Dabei zeigen:
- Fig. 1: eine dreidimensionale Ansicht der Trinkvorrichtung;
- Fig. 2: eine Seitenansicht der in Fig. 1 dargestellten Trinkvorrichtung;
- Fig.3: eine Explosionsdarstellung der in den Fig. 1 und 2 dargestellten Trinkvorrichtung;
- Fig. 4: eine Schnittdarstellung der in den Fig. 1 und 2 dargestellten Trinkvorrichtung;
- Fig. 5: eine Schnittdarstellung der beiden Adapterteile des Adapterelements;
- Fig. 6: eine dreidimensionale Darstellung der in Fig. 5 dargestellten zwei Adapterteile des Adapterelements;
- Fig. 7: das zusammengesetzte Adapterelement;
- Fig. 8: eine Schnittdarstellung entsprechend der Darstellung nach Fig. 4 einer weiteren Ausführungsform der Erfindung; und
- Fig. 9: eine Schnittdarstellung durch einen Aromabehälter.

### Beschreibung bevorzugter Ausführungsformen

In den nachfolgend erläuterten Ausführungsbeispielen werden jeweils dieselben Bauelemente mit denselben Referenzziffern bezeichnet werden und, wo erforderlich, nur auf die spezifischen Unterschiede zwischen den einzelnen Ausführungsformen eingegangen werden.

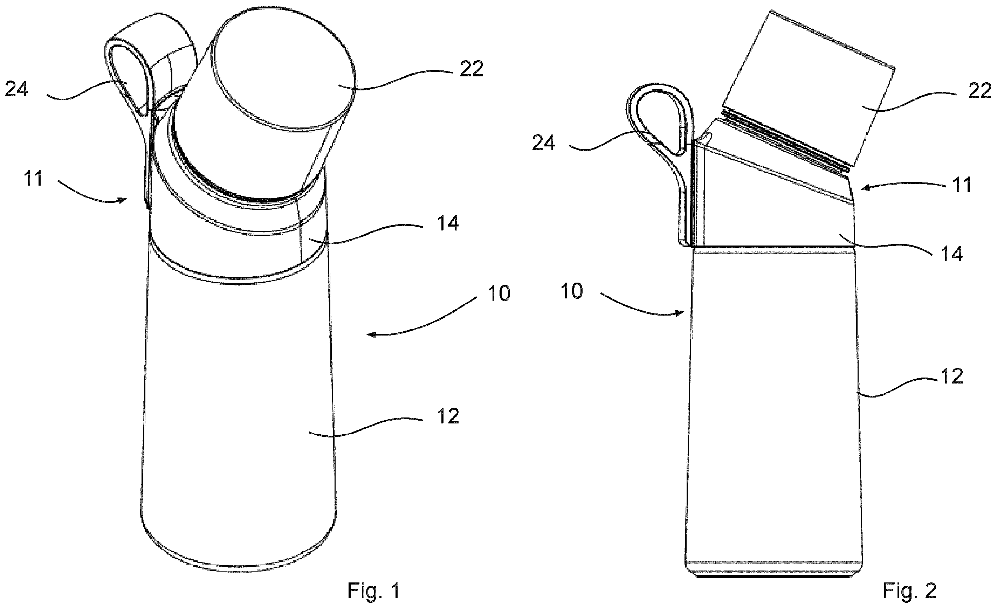

In den Fig. 1 und 2 ist eine erfindungsgemäße Trinkvorrichtung dargestellt. Die Trinkvorrichtung 10 umfasst dabei einen Vorratsbehälter 12 für Trinkflüssigkeit, einen erfindungsgemäßen Aufsatz 11 sowie einen Deckel 22. Am Aufsatz 11 ist eine Trageschlaufe 24 angebracht. In der Außenansicht mit aufgeschraubtem oder aufgestecktem Deckel ist von dem Aufsatz 11 nur das Adapterelement 14 sichtbar. Es ist ersichtlich, dass der Deckel 22 in Bezug auf seine Rotationsachse gegenüber der Längsachse des Vorratsbehälters 12 für Trinkflüssigkeit winklig angeordnet ist, wobei der Winkel mindestens 20° beträgt. Dies winklige Anordnung wird durch die Geometrie des Aufsatzes 11 und insbesondere des Adapterelements 14 erzeugt. Das Adapterelement 14 weist einen Knick auf, so dass die Verbindungsebene zwischen dem Adapterelement 14 und dem Vorratsbehälter 12 winklig zu der Verbindungsebene zwischen dem Adapterelement 14 und dem Deckel 22 steht.

Das Adapterelement 14 ist vorzugsweise aus Kunststoff gefertigt, während der Vorratsbehälter 12 vorzugsweise aus Metall und/oder Glas besteht. Allerdings kann der Vorratsbehälter 12 in gleicher Weise ebenfalls aus Kunststoff hergestellt sein.

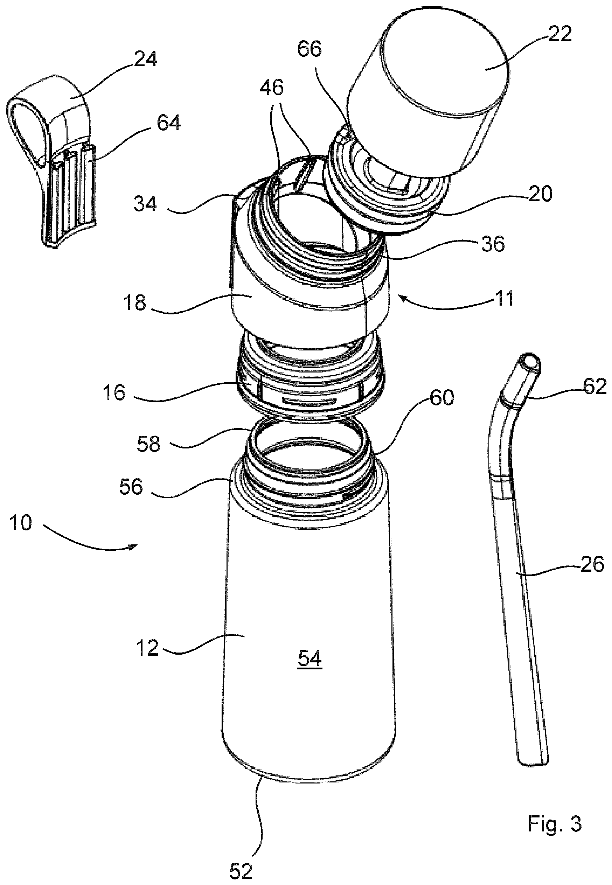

In Fig. 3 sind die in den Fig. 1 und 2 dargestellten einzelnen Elemente der Trinkvorrichtung 10 in einer Explosionsansicht gezeigt. Der Vorratsbehälter 12 für Trinkflüssigkeit weist einen im Wesentlichen ringförmigen Boden 52 sowie eine Seitenwand 54 auf, die sich von dem Boden 52 nach oben erstreckt. Am oberen Ende der Seitenwand befindet sich eine Oberseite 56, die sich von der Seitenwand 54 im Wesentlichen horizontal, radial nach innen erstreckt und von der sich eine ringförmige Einfüllöffnung 58 nach oben erstreckt, deren Außenwand mit einem Befestigungsgewinde 60 versehen ist. Anstelle des Befestigungsgewindes 60 könnte aber in gleicher Weise eine bajonettartige Struktur oder eine geeignete Einrasteinrichtung vorgesehen sein.

Auf das Befestigungsgewinde 60 ist der erfindungsgemäße Aufsatz 11 aufschraubbar. Dazu ist ein Adapterelement 14, das in den Fig. 1 und 2 dargestellt ist, vorgesehen, das ein erstes Adapterteil 16 sowie ein zweites Adapterteil 18 umfasst. Das erste Adapterteil 16 ist dabei so gestaltet, dass es auf das Befestigungsgewinde 60 des Vorratsbehälters 12 aufschraubbar ist, und darüber hinaus mit dem zweiten Adapterteil 18 verbindbar ist. Das zweite Adapterteil 18 ist gekröpft, **d.h.** es weist einen Knick auf, so dass die das erste Adapterteil 16 aufnehmende zweite Öffnung in einer Ebene liegt, die in einem Winkel zu einer ersten Öffnung angeordnet ist, die der Anbringung eines Kopfteils 20 dient.

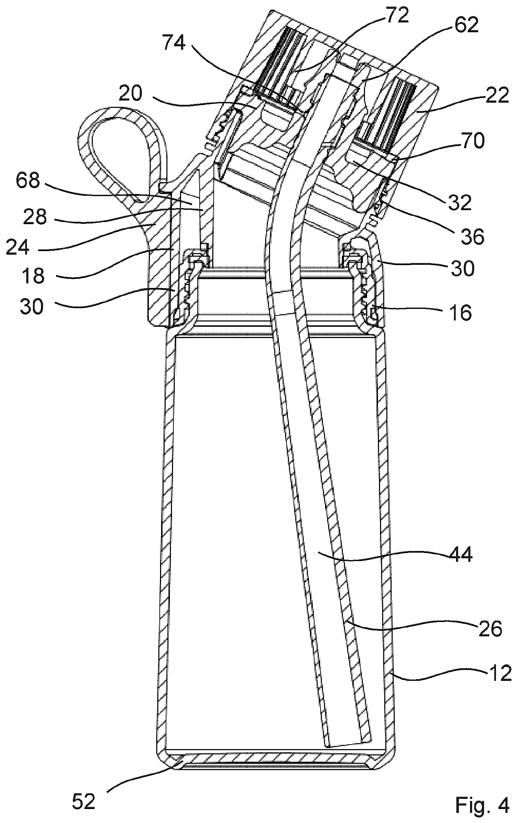

Das Kopfteil 20 ist, wie in der Schnittdarstellung nach Fig. 4 ersichtlich ist, mit einem Mundstück 62 versehen sowie mit einer geeigneten Aufnahmegeometrie für einen Aromabehälter 32. Darüber hinaus ist das Kopfteil 20 so gestaltet, dass ein Trinkhalm 26 an diesem befestigbar ist und bevorzugt in das Kopfteil eingesteckt werden kann.

Der Trinkhalm 26 weist einen in Längsrichtung gekrümmten Verlauf auf und besitzt darüber hinaus zumindest im Bereich des Befestigungsendes 62, mit dem der Trinkhalm 26 am Kopfteil 20 befestigbar ist, eine Außenkontur, die im Zusammenwirken mit einer entsprechend geformten Aufnahmegeometrie im Kopfteil nur eine einzige Orientierung in Bezug auf die Rotationsachse gestattet.

An dem zweiten Adapterteil ist eine Aufnahmeeinrichtung 34 für eine Trageschlaufe vorgesehen, wobei die Trageschlaufe 24 mit einer komplementär geformten Befestigungsgeometrie 64 versehen ist, die so mit der Aufnahmeeinrichtung 34 zusammenwirkt, dass die Befestigungsschlaufe in einer Richtung bis zu einem Anschlag einschiebbar ist und in dieser Position formschlüssig gehalten wird.

Im Bereich der ersten Öffnung 38 des zweiten Adapterteils 18 sind Führungselemente 46 vorgesehen, die mit einer geeigneten Aufnahmegeometrie (in Fig. 3 nicht dargestellt) des Kopfteils zusammenwirken und auf diese Weise nur eine einzige, vordefinierte Orientierung des Kopfteils 20 relativ zum zweiten Adapterteil 18 erlauben. Eine eindeutige Orientierung des Kopfteils relativ zum Adapterelement ist wichtig, da die Trinkvorrichtung eine Druckausgleichsöffnung 66 aufweist, die während des Trinkvorgangs oben angeordnet sein sollte und dazu dient, dass Umgebungsluft beim Trinkvorgang in die Trinkvorrichtung einströmen kann, um das Volumen der aus der Trinkvorrichtung gesogenen Trinkflüssigkeit zu ersetzen.

Das zweite Adapterteil 18 ist im Bereich der ersten Öffnung mit einem Außengewinde 36 versehen, das mit einem in Fig. 3 nicht dargestellten Innengewinde im Deckel 22 zusammenwirkt, um den Deckel 22 auf den Aufsatz 11 aufzuschrauben.

Fig. 4 zeigt eine Schnittdarstellung der in Explosionsdarstellung in Fig. 3 dargestellten Trinkvorrichtung. In Fig. 4 ist die Aufnahme des Aromabehälters 32 im Kopfteil 20 sowie dessen im Schnitt ringförmige Geometrie dargestellt, wobei allerdings die Geometrie des Aromabehälters 32 von einer exakten Kreisringgeometrie abweicht. Darüber hinaus ist erkennbar, dass das zweite Adapterteil 18 doppelwandig ausgeführt ist und eine Innenwand 28 sowie eine Außenwand 30 umfasst. Durch die Doppelwandigkeit des zweiten Adapterteils 18 wird ein Hohlraum 68 gebildet, der die Isolierwirkung des zweiten Adapterteils 18 verbessert und auf diese Weise die Abkühlung warmer Trinkflüssigkeit oder das Erwärmen kalter Trinkflüssigkeit verzögert. Der Hohlraum 68 kann auch ausgeschäumt sein, um die Isolierwirkung zu verbessern. Die Doppelwandigkeit dient aber auch dazu, bei gleichen mechanischen Eigenschaften die Wanddicke verringern zu können und auf diese Weise den Materialeinsatz zur Herstellung des Aufsatzes zu verringern.

Der Hohlraum 68 kann auch dazu dienen, weitere Elemente aufzunehmen, wie Sensoren, Batterien, Dioden, z.B. zur Aussendung von UV-Licht, oder aber auch Heiz- oder Kühlelemente.

Wie ebenfalls in Fig. 4 ersichtlich ist, ist im Deckel ein ringförmiger Absatz 70 geformt, der im aufgeschraubten Zustand des Deckels 22 gegen das Kopfteil 20 abdichtet. Darüber hinaus ist im Deckel ein Deaktivierungsring 72 eingeformt, der den Aromabehälter 32 beim Schließen des Deckels nach unten relativ zum Kopfteil 20 verschiebt und auf diese Weise in eine Ruheposition bringt, in der der Aromabehälter 32 abgedichtet ist, weil eine Luftaustrittsöffnung aus dem Aromabehälter 32 nicht mit einem Luftkanal 74 ausgerichtet ist, der dazu dient, in der aktivierten Position des Aromabehälters die aus dem Aromabehälter austretende, aromatisierte Luft in den Transportkanal 44 für Trinkflüssigkeit zu fördern.

Wie weiterhin in Fig. 4 dargestellt ist, ist die Verbindung in axialer Richtung zwischen dem ersten Adapterteil 16 und dem zweiten Adapterteil 18 eine reibschlüssige Verbindung. In gleicher Weise könnte hier aber auch eine Einschnappverbindung hergestellt werden, die beispielsweise erst wieder gelöst werden kann, wenn beispielsweise durch Druck auf eine bestimmte Stelle am zweite Adapterelement die Arretierung wieder gelöst wird.

Wie ebenfalls in Fig. 4 dargestellt ist, ist der Trinkhalm 26 so weit in das Kopfteil 20 eingeschoben, dass sich ein Ende des Trinkhalms 26 bis in unmittelbarer Nähe zum Boden 52 des Vorratsbehälters 12 für Trinkflüssigkeit erstreckt.

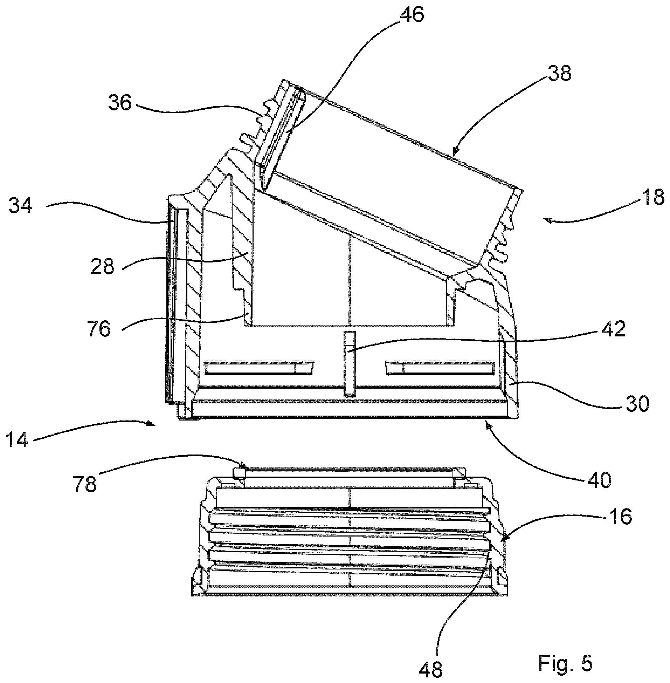

Ein konkretes Ausführungsbeispiel für die Gestaltung des Adapterelements 14 bestehend aus dem ersten Adapterteil 16 und dem zweiten Adapterteil 18 ist in Fig. 5 dargestellt. Dabei sind die Innenwand 28 des zweiten Adapterteils, die Außenwand 30 des zweiten Adapterteils, die Aufnahmeeinrichtung 34 zur Anbringung der Trageschlaufe, das mindestens eine Führungselement 46 zur Positionierung des Kopfteils und das Außengewinde 36 im Bereich der ersten Öffnung 38 des zweiten Adapterteils detaillierter als in den vorangehenden Ausführungsformen dargestellt. In gleicher Weise ist auch die winklige Anordnung der ersten Öffnung 38 relativ zur zweiten Öffnung 40 dargestellt. Das Zentrier- und Positionierelement 42, das eine ungewollte Rotation zwischen dem ersten Adapterteil 16 und dem zweiten Adapterteil 18 im verhindert, kann dabei als einfache, in Längsrichtung verlaufende Rippe ausgestaltet sein, die mit einer entsprechend geformten Nut im ersten Adapterteil 16 zusammenwirkt.

Die Innenwand 28 des zweiten Adapterteils 18 ist mit einem Führungsbund 76 versehen, der im montierten Zustand des Adapterelements 14 in eine obere Öffnung 78 des ersten Adapterteils 16 einrückt, wie auch in Fig. 4 ersichtlich ist, und sowohl die Stabilität des Adapterelements 14 als auch die korrekte Positionierung des ersten Adapterteils 16 relativ zum zweiten Adapterteil 18 sicherstellt.

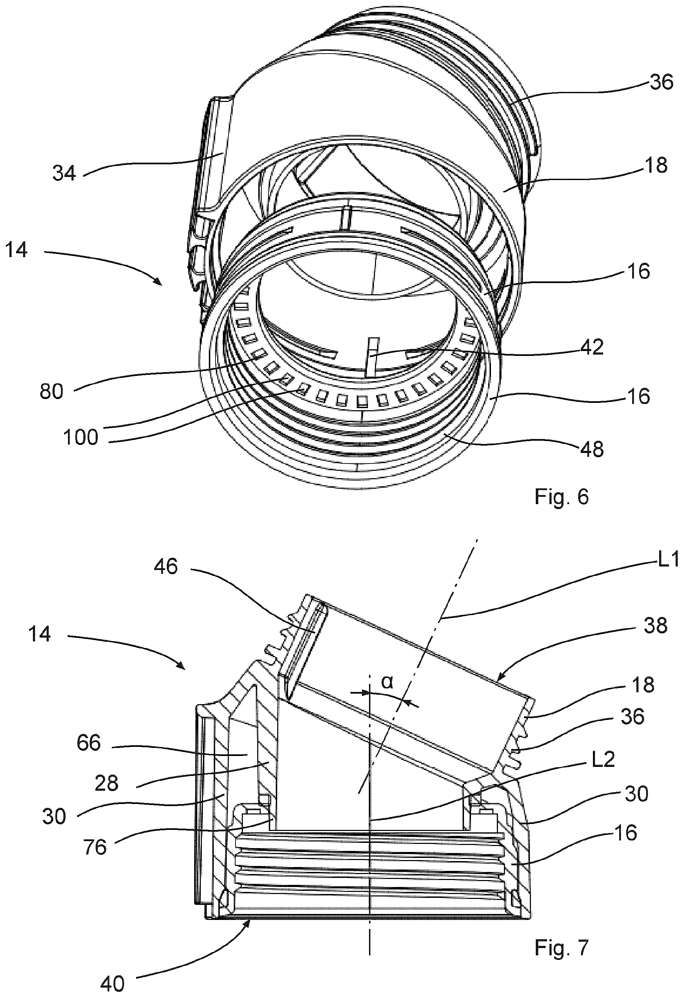

In den Fig. 6 und 7 ist das Adapterelement 14 noch einmal dargestellt, wobei die Fig. 6 einer dreidimensionalen Darstellung der Fig. 5 entspricht, und in Fig. 7 eine Schnittdarstellung das Adapterelements 14 gezeigt ist, in der das ersten Adapterteil 16 mit dem zweiten Adapterteil 18 verbunden ist, also im montierten Zustand des Adapterelements. Dabei ist in Fig. 7 auch der Winkel α dargestellt, in dem die Längsachsen L1 und L2 zueinander stehen, wobei die erste Längsachse L1 senkrecht zur ersten Öffnung 38 steht, und die zweite Längsachse L2, die senkrecht zur zweiten Öffnung 40 steht, wobei die zweite Längsachse L2 auch der Längsachse des ersten Adapterteils 16 entspricht. Der Winkel α zwischen der ersten Längsachse L1 und der zweiten Längsachse L2 beträgt mindestens 20° und liegt bevorzugt zwischen 20° und 40°.

In Fig. 7 ist darüber hinaus das Zusammenwirken des Führungsbunds 76 des zweiten Adapterteils 18 mit der oberen Öffnung des ersten Adapterteils 16 dargestellt.

In Fig. 6 ist eine mögliche Formgebung der Aufnahmeeinrichtung 34 zur Anbringung der Trageschlaufe dargestellt, die eine formschlüssige Geometrie zum Einschieben einer entsprechend geformten Befestigungsgeometrie 64 (siehe Fig. 3) an der Trageschlaufe 24 erlaubt. Darüber hinaus ist in Fig. 6 eine Dichtungsaufnahme 80 dargestellt, die dazu dient, einen elastischen Dichtring (nicht dargestellt) aufzunehmen, der beim Aufschrauben des ersten Adapterteils 16 auf den Vorratsbehälter 12 im Gewindebereich zwischen dem Vorratsbehälter 12 und dem Adapterelement 14 abdichtet.

Über den Umfang der ringförmigen Dichtungsaufnahme 80 verteilt sind in der Dichtungsaufnahme zahlreiche Vertiefungen 100 vorgesehen, die sich in Montageposition zwischen dem ersten Adapterteil 16 und einer in die Dichtungsaufnahme 80 eingelegten, ringförmigen Dichtung befinden. Die in den Vertiefungen 100 befindlichen Luftpolster werden dazu verwendet, um Fertigungstoleranzen auszugleichen und die Dichtwirkung zu verbessern, indem beim Aufschrauben des Adapterelements auf den Vorratsbehälter für Trinkflüssigkeit die Luft in den Vertiefungen 100 komprimiert wird. Das Vorsehen von zahlreichen Vertiefungen 100 erhöht zudem die Lebensdauer des Dichtelements.

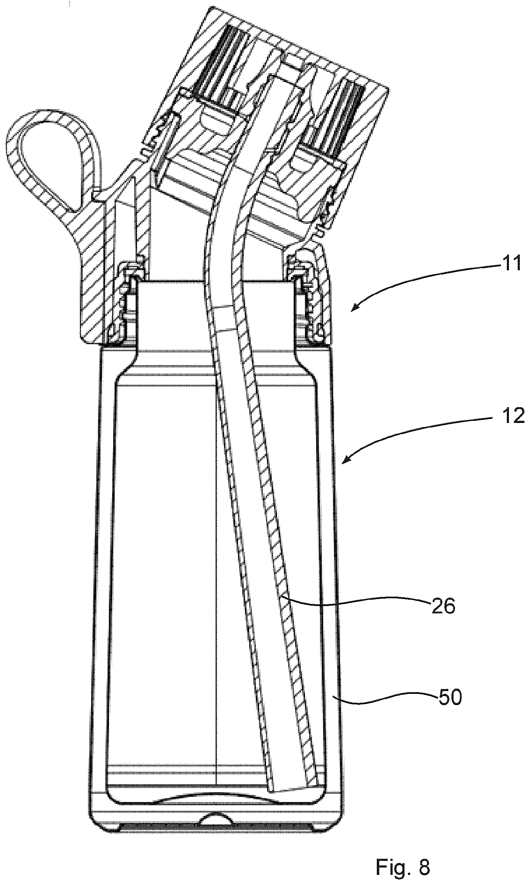

Die Ausführungsform nach Fig. 8 entspricht weitestgehend derjenigen nach Fig. 4, so dass für eine Erläuterung des Aufsatzes 11 auf die vorhergehenden Ausführungsformen verwiesen werden kann. Der Unterschied zu den vorangehend erläuterten Ausführungsformen besteht allerdings darin, dass der Vorratsbehälter 12 für Trinkflüssigkeit ein doppelwandiges Gefäß 50 ist, wie es als Isoliergefäß oder Thermosgefäß bezeichnet wird. Der doppelwandige Vorratsbehälter 50 besteht vorzugsweise aus Metall, während der Aufsatz 11 aus Kunststoff gefertigt ist. Allerdings können auch andere Materialpaarungen vorgesehen sein und beispielsweise der Vorratsbehälter auch aus Glas bestehen oder aber einer Kombination aus einer Außenschale aus Metall und einer Innenschale aus verspiegeltem Glas.

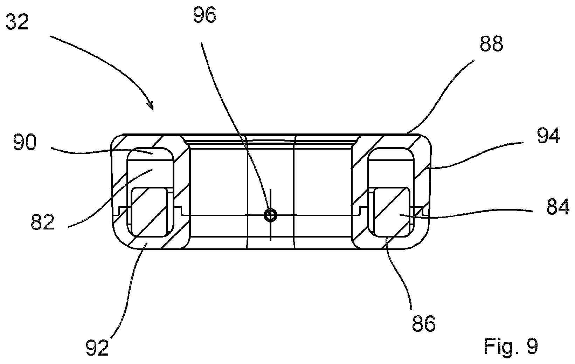

In Fig. 9 ist eine Schnittdarstellung des Aromabehälters 32 dargestellt. Der Aromabehälter 32 besitzt dabei eine Aromakammer 82, die durch einen inneren Hohlraum 68 im Aromabehälter 32 gebildet ist. In dieser Aromakammer 82 befindet sich eine Trägersubstanz 84, die in der in Fig. 8 dargestellten Betriebsposition auf der Unterseite 86 der Aromakammer 82 aufliegt. Zwischen der Trägersubstanz 84 und der Oberseite 88 der Aromakammer 82 ist ein Kopfraum 90 gebildet, in dem die Aromakammer 82 nicht mit Trägersubstanz 84 gefüllt ist. Das Vorsehen des Kopfraums 90 besitzt die Funktion, ein besseres Anreichern der Luft sowie eine Homogenisierung der den Aromabehälter durchströmenden Luft mit Aromasubstanz zu ermöglichen. Der Kopfraum 90 besitzt dabei eine Höhe, die geringer als 50% der Höhe der Aromakammer 82 ist. Es ist allerdings bevorzugt, dass die Trägersubstanz 84 mindestens 80% der Höhe der Aromakammer 90 einnimmt.

Der Aromabehälter 32 ist aus einer unteren Schale 92 sowie einer oberen Schale 94 gebildet. Die Lufteintrittsöffnung, die in der Schnittdarstellung nach Fig. 9 nicht dargestellt ist, befindet sich in der unteren Schale 92, während eine Luftaustrittsöffnung 96 in einer inneren Seitenwand des im Wesentlichen ringförmigen Aromabehälters gebildet ist. Aus fertigungstechnischen Gründen hat es sich dabei als vorteilhaft erwiesen, die Luftaustrittsöffnung 96 an einer Nahtstelle zwischen der unteren Schalte 92 und der oberen Schale 94 anzuordnen, was bei einer Massenfertigung der Aromabehälter im Spritzgussverfahren vorteilhaft ist.

Die Trägersubstanz 84 ist poröses Aromaträgermaterial, beispielsweise ein Vliesmaterial, dessen Porosität bevorzugt zwischen 70% und 93% und bevorzugt zwischen 70% und 80% beträgt. Der Durchmesser der in Fig. 9 nicht dargestellten Lufteintrittsöffnung in der unteren Wand liegt zwischen 1.1mm bis **1.3mm.**

Die untere Schale 92 und die obere Schale 94 können per Ultraschallschweißverfahren miteinander verbunden werden und werden damit außer im Bereich der Luftaustrittsöffnung 96 umlaufend dicht verbunden, so dass ein potentielles Eindringen von Luft in den Aromabehälter sowie ein ungewünschtes Austreten von Aromasubstanz aus dem Aromabehälter 32 vermieden werden kann.

### Bezugszeichenliste

- 10: Trinkvorrichtung
- 11: Aufsatz
- 12: Vorratsbehälter für Trinkflüssigkeit
- 14: Adapterelement
- 16: erstes Adapterteil
- 18: zweites Adapterteil
- 20: Kopfteil
- 22: Deckel
- 24: Trageschlaufe
- 26: Trinkhalm
- 28: Innenwand des zweiten Adapterteils
- 30: Außenwand des zweiten Adapterteils
- 32: Aromabehälter
- 34: Aufnahmeeinrichtung zur Aufnahme der Trageschlaufe
- 36: Außengewinde im Bereich der ersten Öffnung
- 38: erste Öffnung
- 40: zweite Öffnung
- 42: Zentrier- und Positioniereinrichtung
- 44: Transportkanal für Trinkflüssigkeit
- 46: Führungselement
- 48: Innengewinde des ersten Adapterteils
- 50: doppelwandiger Vorratsbehälter
- 52: Boden
- 54: Seitenwand
- 56: Oberseite
- 58: Einfüllöffnung
- 60: Befestigungsgewinde
- 62: Befestigungsende
- 64: Befestigungsgeometrie
- 66: Druckausgleichsöffnung
- 68: Hohlraum
- 70: Absatz
- 72: Deaktivierungsring
- 74: Luftkanal
- 76: Führungsbund
- 78: obere Öffnung
- 80: Dichtungsaufnahme
- 82: Aromakammer
- 84: Trägersubstanz
- 86: Unterseite der Aromakammer
- 88: Oberseite der Aromakammer
- 90: Kopfraum
- 92: untere Schale
- 94: obere Schale
- 96: Luftaustrittsöffnung
- 98: innere Seitenwand
- 100: Vertiefungen

## Patentansprüche

1. Aufsatz für eine Trinkvorrichtung zur retronasalen Aufnahme einer Aromasubstanz, umfassend:
- ein Adapterelement (14) zur flüssigkeitsdichten Anbringung an einem Vorratsbehälter (12) für Trinkflüssigkeit;
- ein mit einem Mundstück (62) versehenes Kopfteil (20) zur lösbaren Befestigung an dem Adapterelement (14);
- einen luftdurchströmbaren Aromabehälter (32), der am Kopfteil (20) befestigbar ist; und
- einen Transportkanal (44) für Trinkflüssigkeit, der am Kopfteil (20) befestigbar ist;
**dadurch gekennzeichnet, dass**:
- das Adapterelement (14) ein erstes Adapterteil (16) sowie ein zweites Adapterteil (18) umfasst; und
- das erste Adapterteil (16) zur Anbringung an dem Vorratsbehälter (12) sowie zur Anbringung an dem zweiten Adapterteil (18) angepasst ist; und
- das zweite Adapterteil (18) eine erste Öffnung (36) mit einer ersten Längsachse (L1) sowie eine zweite Öffnung (40) mit einer zweiten Längsachse (L2) aufweist; und
- die erste Längsachse (L1) in einem Winkel zur zweiten Längsachse (L2) steht, der mindestens 20° beträgt.

2. Aufsatz für eine Trinkvorrichtung nach Anspruch 1,
**dadurch gekennzeichnet, dass**
der Aromabehälter (32) einen von einer Behälterwand umschlossenen, inneren Hohlraum (68), eine Lufteintrittsöffnung durch die Behälterwand und eine Luftaustrittsöffnung (96) durch die Behälterwand umfasst.

3. Aufsatz für eine Trinkvorrichtung nach Anspruch 2,
**dadurch gekennzeichnet, dass**
das Kopfteil (20) einen Luftkanal (74) aufweist, dessen zweites Ende in Strömungsverbindung mit einem Innenraum im Transportkanal (44) für Trinkflüssigkeit steht, und dessen erstes Ende so angeordnet ist, dass der Aromabehälter (32) in eine Position bewegbar ist, in der die Luftaustrittsöffnung (96) mit dem ersten Ende des Luftkanals (74) in Strömungsverbindung steht.

4. Aufsatz für eine Trinkvorrichtung nach einem der Ansprüche 1 bis 3,
**dadurch gekennzeichnet, dass**
das zweite Adapterteil (18) zumindest bereichsweise mehrwandig, insbesondere doppelwandig ist.

5. Aufsatz für eine Trinkvorrichtung nach Anspruch 4,
**dadurch gekennzeichnet, dass**
in einem Hohlraum (68) zwischen einer Innenwand (28) und einer Außenwand (30) des zweiten Adapterteils (18) ein UV-Strahlung emittierendes Bauelement platzierbar ist.

6. Aufsatz für eine Trinkvorrichtung nach einem der vorhergehenden Ansprüche,
**dadurch gekennzeichnet, dass**
der Transportkanal (44) für Trinkflüssigkeit eine Längsachse besitzt, die einen gekrümmten Verlauf hat.

7. Aufsatz für eine Trinkvorrichtung nach einem der vorhergehenden Ansprüche,
**dadurch gekennzeichnet, dass**
der Transportkanal für Trinkflüssigkeit ein Trinkhalm (44) ist, dessen Außenquerschnitt zumindest im Bereich der Befestigung am Kopfteil (20) so gestaltet ist, dass der Trinkhalm (44) nur in einer einzigen Rotationsposition in Bezug auf eine Längsachse des Transportkanals für Trinkflüssigkeit am Kopfteil (20) befestigbar ist.

8. Aufsatz für eine Trinkvorrichtung nach einem der vorhergehenden Ansprüche,
**dadurch gekennzeichnet, dass**
der Aromabehälter (32) im Wesentlichen ringförmig ist mit einer radial äußeren Begrenzungswand und einer radial inneren Begrenzungswand (98), wobei die radial innere Begrenzungswand (98) einen Raum umschließt, dessen Querschnittsfläche eine von einer kreisrunden Form abweichende Geometrie besitzt.

9. Aufsatz für eine Trinkvorrichtung nach einem der vorhergehenden Ansprüche,
**dadurch gekennzeichnet, dass**
das Adapterelement (14) eine Aufnahmeeinrichtung (34) zur Anbringung einer Trageschlaufe (24) aufweist.

10. Aufsatz für eine Trinkvorrichtung nach einem der vorhergehenden Ansprüche,
**dadurch gekennzeichnet, dass**
das Adapterelement (14) sowie das Kopfteil (20) aus Kunststoff bestehen.

11. Aufsatz für eine Trinkvorrichtung nach einem der vorhergehenden Ansprüche,
**dadurch gekennzeichnet, dass**
das zweite Adapterteil (18) in Bereich der ersten Öffnung (36) mindestens ein erstes Positionierelement (42) aufweist, das mit einem zweiten Positionierelement zusammenwirkt, das am Kopfteil (20) vorgesehen ist, so dass das Kopfteil (20) nur in einer definierten Rotationsposition relativ zur ersten Längsachse (L1) an dem zweiten Adapterelement (16) anbringbar ist.

12. Trinkvorrichtung zur retronasalen Aufnahme einer Aromasubstanz, umfassend
- einen Vorratsbehälter (12) für Trinkflüssigkeit; und
- einen an dem Vorratsbehälter (12) für Trinkflüssigkeit befestigten Aufsatz (11) nach einem der vorhergehenden Ansprüche.

13. Trinkvorrichtung nach Anspruch 12,
**dadurch gekennzeichnet, dass**
der Vorratsbehälter (12) für Trinkflüssigkeit aus einem anderen Material besteht als das Adapterelement (14), vorzugsweise das Adapterelement (14) aus Kunststoff besteht und der Vorratsbehälter (12) für Trinkflüssigkeit aus Metall besteht.

14. Trinkvorrichtung nach Anspruch 12 oder Anspruch 13,
**dadurch gekennzeichnet, dass**
der Vorratsbehälter zumindest bereichsweise doppelwandig ist.

## Claims

1. Attachment for a drinking device for the retronasal perception of an aroma substance, comprising:
- an adapter element (14) to be fluid-tightly attached to a storage container (12) for drinking liquid;
- a head part (20) provided with a mouthpiece (62) and to be detachably fastened to the adapter element (14);
- an aroma container (32) through which air can flow and which can be fastened to the head part (20); and
- a transport channel (44) for drinking liquid, which can be fastened to the head part (20);
**characterized in that**
- the adapter element (14) comprises a first adapter part (16) and a second adapter part (18); and
- the first adapter part (16) is adapted to be attached to the storage container (12) and to be attached to the second adapter part (18); and
- the second adapter part (18) comprises a first opening (36) having a first longitudinal axis (L1) and a second opening (40) having a second longitudinal axis (L2); and
- the first longitudinal axis (L1) is at an angle to the second longitudinal axis (L2), which is at least 20°.

2. Attachment for a drinking device according to claim 1,
**characterized in that**
the aroma container (32) has an inner cavity (68) enclosed by a container wall, an air inlet opening through the container wall and an air outlet opening (96) through the container wall.

3. Attachment for a drinking device according to claim 2,
**characterized in that**
the head part (20) has an air channel (74), the second end of which is in flow connection with an interior space in the transport channel (44) for drinking liquid, and the first end of which is arranged in such a way that the aroma container (32) can be moved into a position in which the air outlet opening (96) is in flow connection with the first end of the air channel (74).

4. Attachment for a drinking device according to one of claims 1 to 3,
**characterized in that**
the second adapter part (18) is at least sectionally multi-walled, in particular double-walled.

5. Attachment for a drinking device according to claim 4,
**characterized in that**
a UV-radiation-emitting component can be placed in a cavity (68) between an inner wall (28) and an outer wall (30) of the second adapter part (18).

6. Attachment for a drinking device according to one of the preceding claims,
**characterized in that**
the transport channel (44) for drinking liquid has a longitudinal axis which extends in a curved manner.

7. Attachment for a drinking device according to one of the preceding claims,
**characterized in that**
the transport channel for drinking liquid is a drinking straw (44), the outer cross-section of which is configured in such a way, at least in the area where it is fastened to the head part (20), that the drinking straw (44) can only be fastened to the head part (20) in a single rotational position in relation to a longitudinal axis of the transport channel for drinking liquid.

8. Attachment for a drinking device according to one of the preceding claims,
**characterized in that**
the aroma container (32) is essentially annular with a radially outer boundary wall and a radially inner boundary wall (98), wherein the radially inner boundary wall (98) encloses a space, the cross-sectional area of which has a geometry deviating from a circular shape.

9. Attachment for a drinking device according to one of the preceding claims,
**characterized in that**
the adapter element (14) has a receiving means (34) for attaching a carrying strap (24).

10. Attachment for a drinking device according to one of the preceding claims,
**characterized in that**
the adapter element (14) and the head part (20) are made of plastic.

11. Attachment for a drinking device according to one of the preceding claims,
**characterized in that**
in the area of the first opening (36) the second adapter part (18) has at least one first positioning element (42) interacting with a second positioning element provided at the head part (20), so that the head part (20) can only be attached to the second adapter part (18) in a defined rotational position relative to the first longitudinal axis (L1).

12. Drinking device for the retronasal perception of an aroma substance, comprising
- a storage container (12) for drinking liquid; and
- -an attachment (11) fastened to the storage container (12) for drinking liquid according to one of the preceding claims.

13. Drinking device according to claim 12,
**characterized in that**
the storage container (12) for drinking liquid is made of a different material than the adapter element (14), preferably the adapter element (14) is made of plastic and the storage container (12) for drinking liquid is made of metal.

14. Drinking device according to claim 12 or claim 13,
**characterized in that**
the storage container is at least sectionally double-walled.

## Revendications

1. Embout pour dispositif de boisson pour absorption rétro-nasale d'une substance aromatique, comprenant :
- un élément adaptateur (14) destiné à être fixé de manière étanche sur un réservoir (12) pour du liquide à boire ;
- une partie supérieure (20) munie d'un embout buccal (62) destinée à être fixée de manière amovible à l'élément adaptateur (14) ;
- un réservoir d'arôme (32) perméable à l'air, pouvant être fixé à la partie supérieure (20) ; et
- un canal de transport (44) pour du liquide à boire, pouvant être fixé à la partie supérieure (20) ;
**caractérisé en ce que** :
- l'élément adaptateur (14) comprend une première partie d'adaptateur (16) ainsi qu'une deuxième partie d'adaptateur (18) ; et
- la première partie d'adaptateur (16) est adaptée pour être fixée au réservoir (12) ainsi que pour être fixée à la deuxième partie d'adaptateur (18) ; et
- la deuxième partie d'adaptateur (18) a une première ouverture (36) avec un premier axe longitudinal (L1) ainsi qu'une deuxième ouverture (40) avec un deuxième axe longitudinal (L2) ; et
- le premier axe longitudinal (L1) forme avec le deuxième axe longitudinal (L2) un angle d'au moins 20°.

2. Embout pour dispositif de boisson selon la revendication 1,
**caractérisé en ce que**
le réservoir d'arôme (32) comprend une cavité interne (68) entourée par une paroi de récipient, une ouverture d'entrée d'air à travers la paroi de récipient et une ouverture de sortie d'air (96) à travers la paroi de récipient.

3. Embout pour dispositif de boisson selon la revendication 2,
**caractérisé en ce que**
la partie supérieure (20) a un canal d'air (74) dont la deuxième extrémité est en communication fluidique avec un espace intérieur du canal de transport (44) pour du liquide à boire, et dont la première extrémité est agencée de telle sorte que le réservoir d'arôme (32) puisse être déplacé dans une position dans laquelle l'ouverture de sortie d'air (96) est en communication fluidique avec la première extrémité du canal d'air (74).

4. Embout pour dispositif de boisson selon l'une des revendications 1 à 3,
**caractérisé en ce que**
la deuxième partie d'adaptateur (18) est au moins par endroits à paroi multiple, en particulier à double paroi.

5. Embout pour dispositif de boisson selon la revendication 4,
**caractérisé en ce que**
un composant émettant du rayonnement UV peut être placé dans une cavité (68) entre une paroi intérieure (28) et une paroi extérieure (30) de la deuxième partie d'adaptateur (18).

6. Embout pour dispositif de boisson selon l'une des revendications précédentes,
**caractérisé en ce que**
le canal de transport (44) pour du liquide à boire possède un axe longitudinal qui a un tracé courbe.

7. Embout pour dispositif de boisson selon l'une des revendications précédentes,
**caractérisé en ce que**
le canal de transport pour du liquide à boire est une paille (44) dont la section transversale extérieure, au moins dans la zone de fixation à la partie supérieure (20), est conçue de telle sorte que la paille (44) ne puisse être fixée à la partie supérieure (20) que dans une seule position de rotation par rapport à un axe longitudinal du canal de transport pour du liquide à boire.

8. Embout pour dispositif de boisson selon l'une des revendications précédentes,
**caractérisé en ce que**
le réservoir d'arôme (32) est essentiellement annulaire avec une paroi de délimitation radiale extérieure et une paroi de délimitation radiale intérieure (98), la paroi de délimitation radiale intérieure (98) entourant un espace dont la section transversale a une géométrie s'écartant d'une forme circulaire.

9. Embout pour dispositif de boisson selon l'une des revendications précédentes,
**caractérisé en ce que**
l'élément adaptateur (14) a un dispositif de fixation (34) destiné à la mise en place d'une dragonne (24).

10. Embout pour dispositif de boisson selon l'une des revendications précédentes,
**caractérisé en ce que**
l'élément adaptateur (14) ainsi que la partie supérieure (20) sont en matière plastique.

11. Embout pour dispositif de boisson selon l'une des revendications précédentes,
**caractérisé en ce que**
la deuxième partie d'adaptateur (18) a dans la zone de la première ouverture (36), au moins un premier élément de positionnement (42) qui fonctionne avec un deuxième élément de positionnement prévu sur la partie supérieure (20), de sorte que la partie supérieure (20) ne puisse être fixée au deuxième élément adaptateur (16) que dans une position de rotation définie par rapport au premier axe longitudinal (L1).

12. Dispositif de boisson pour absorption rétro-nasale d'une substance aromatique, comprenant
- un réservoir (12) pour du liquide à boire ; et
- un embout (11) fixé au réservoir (12) pour du liquide à boire, selon l'une des revendications précédentes.

13. Dispositif de boisson selon la revendication 12,
**caractérisé en ce que**
le réservoir (12) pour du liquide à boire est constitué d'un matériau différent de celui de l'élément adaptateur (14), de préférence l'élément adaptateur (14) est en matière plastique et le réservoir (12) pour du liquide à boire est en métal.

14. Dispositif de boisson selon la revendication 12 ou la revendication 13,
**caractérisé en ce que**
le réservoir est à double paroi au moins par endroits.
